# EUROPEAN PATENT APPLICATION

(11) **EP 4 837 624 A1**
(43) Date of publication of application: **11.11.2026**
(21) Application number: 24915482.4
(22) Date of filing: 17.12.2024
(51) Int. Cl.: A61N 1/40, A61N 1/08, A61N 1/36, A61N 1/32, A61N 1/06

(54) **TREATMENT APPARATUS USING RF ENERGY, METHOD FOR CONTROLLING SAME, AND TREATMENT METHOD USING SAME**

(30) Priority: 04.01.2024 KR 20240001508
(71) Applicant: Lutronic Corporation, Goyang-si, Gyeonggi-do 10534 (KR)
(72) Inventor: KO, Kwangchon, Goyang-si, Gyeonggi-do 10394 (KR); LEE, Chang Sub, Goyang-si, Gyeonggi-do 10523 (KR)
(74) Representative: Algemeen Octrooi- en Merkenbureau B.V.
(86) International application number: PCT/KR2024/097061
(87) International publication number: WO 2025/147072

(57) **Abstract**

The present disclosure relates to a treatment apparatus using RF energy, a method for controlling the treatment apparatus, and a treatment method using the treatment apparatus. The treatment apparatus using RF energy includes: a handpiece having an electrode configured to transmit RF energy in contact with a skin surface; an RF energy generator configured to generate RF energy that is transmitted to the electrode; a cooler configured to cool the electrode; and a controller configured to control operation of the RF energy generator and the cooler and control the cooler to preliminarily cool the electrode before RF energy is applied to the electrode.

## Description

### TECHNICAL FIELD

The present disclosure relates to a treatment apparatus using RF energy, a method for controlling the treatment apparatus, and a treatment method using the treatment apparatus.

### BACKGROUND

Technologies of transmitting RF energy to tissues to treat lesions have been developed in various ways. In particular, recently, technologies of treating tissues without damaging the skin surface by performing treatment by transmitting RF energy while cooling the skin with electrodes in contact with the skin surface have been developed. Such treatment technologies using RF energy have been disclosed in Korean Patent No. 0706115, etc. However, the treatment technologies using RF energy of the related art have a problem of inconsistent treatment results depending on the treatment environment and the characteristics of patients.

### SUMMARY

An objective of the present disclosure is to provide a treatment apparatus using RF energy, a method for controlling the treatment apparatus, and a treatment method using the treatment apparatus, the treatment apparatus being able to minimize the differences in treatment results that are caused by different treatment environments and different characteristics of patients despite being different.

In order to achieve the objectives of the present disclosure described above, the present disclosure provides a treatment apparatus using RF energy that includes: a handpiece having an electrode configured to transmit RF energy in contact with a skin surface; an RF energy generator configured to generate RF energy that is transmitted to the electrode; a cooler configured to cool the electrode; and a controller configured to control operation of the RF energy generator and the cooler and control the cooler to preliminarily cool the electrode before RF energy is applied to the electrode.

The controller performs control to preliminarily cool the electrode such that the electrode forms a preset reference temperature without RF energy applied. In this case, the preset temperature may be temperature lower than room temperature, and for example, may be a value within a range of 0 ~ 25°C.

The controller may perform control to preliminarily cool the electrode regardless of a time point when the electrode is brought in contact with the skin surface or a time point when RF energy is applied to the electrode.

The controller may control the cooler to cool the electrode while RF energy is applied through the electrode, and the cooler may be controlled such that a process of preliminarily cooling the electrode and a process of cooling the electrode while the RF energy is applied are continuously performed.

As an example, the handpiece further includes a tip module having the electrode disposed on one surface thereof and detachably coupled to an end portion of a main body of the handpiece. When the tip module is coupled to the main body of the handpiece, the controller may control the cooler to automatically preliminarily cool the electrode. In this configuration, the tip module further includes a memory configured to store information of the tip module and the controller performs control to preliminarily cool the electrode on the basis of the information of the tip module stored in the memory.

The cooler includes a refrigerant storage in which a refrigerant is accommodated, a cooling channel at least partially located in the handpiece and configured to form a path through which the refrigerant accommodated in the refrigerant storage is transmitted to the electrode, and a valve configured to adjust the amount of a refrigerant that is delivered through the cooling channel. Further, the valve may be a proportional control valve such that the operation of cooling the electrode is continuously performed.

As an example, the handpiece further includes a contact sensor disposed adjacent to the electrode and configured to sense a contact state of skin of a patient and the electrode. The controller may control the RF generator to transmit the RF energy after a preset time has passed from a time point when the electrode has been brought in contact with a skin surface of a patient.

Further, the controller may control the cooler to preliminarily cool the electrode while a user moves the handpiece to a second position after treating a first position.

Meanwhile, the objectives of the present disclosure described above may be achieved by a method of controlling a treatment apparatus using RF energy, the method including: sensing that a tip module having an electrode configured to transmit RF energy in contact with a skin surface is fastened to an end of a handpiece; preliminarily cooling the electrode by means of a cooler before RF energy is transmitted through the electrode; and applying RF energy generated by an RF energy generator to the cooled electrode.

Further, the objectives of the present disclosure described above may be achieved by a treatment method using RF energy that includes: preliminarily cooling an electrode configured to transmit RF energy in contact with a skin surface to a preset temperature; and applying RF energy by bringing the electrode cooled to the preset temperature in contact with a skin surface.

According to the present disclosure, since treatment is performed with an electrode preliminarily cooled to a preset temperature, it is possible to perform treatment using an electrode having a constant electrical property even though the temperatures of treatment environment and the skin temperatures of patients are different, so uniform treatment is possible.

Further, since a preliminarily cooled electrode is used, treatment can be performed with the skin of a patient at temperature within a predetermined range, so it is possible to perform uniform treatment while preventing damage to the skin surface.

### BRIEF DESCRIPTION OF THE DRAWINGS

FIG. 1 is a perspective view showing a treatment apparatus using RF energy according to an embodiment of the present disclosure.
FIG. 2 is a block diagram showing the main configuration of the treatment apparatus shown in FIG. 1.
FIG. 3 is a perspective view showing the handpiece shown in FIG. 1.
FIG. 4 is an exploded perspective view showing the main configuration of the tip module shown in FIG. 3.
FIG. 5 is a cross-sectional view showing the main configuration of the tip module shown in FIG. 3.
FIG. 6 is a graph showing temperature change of an electrode according to the precooling in FIG. 3.
FIG. 7 is a graph showing cooling and RF application patterns that are applied to an electrode in accordance with a treatment process.
FIG. 8 is a flowchart showing a method for controlling the treatment apparatus using RF energy according to an embodiment.
FIG. 9 is a flowchart showing the detailed steps of the precooling step shown in FIG. 8.
FIG. 10 is a graph showing a cooling pattern and temperature change in the precooling step shown in FIG. 7.
FIG. 11 is a graph exemplarily showing temperature sensed by a temperature sensor.
FIG. 12 is a flowchart showing a method for controlling the treatment apparatus using RF energy according to another embodiment.
FIG. 13 is a flowchart showing a treatment method using the treatment apparatus using RF energy according to another embodiment.

### DETAILED DESCRIPTION

Hereafter, a treatment apparatus using RF energy according to an embodiment of the present disclosure, a method for controlling the treatment apparatus, and a treatment method using the treatment apparatus are described in detail with reference to the drawings. In the following description, the positional relationships of components are principally explained with reference to the drawings. Further, the drawings may simplify the structure of the present disclosure or may be exaggerated, if necessary, for the convenience of description. Accordingly, the present disclosure is not limited thereto and it should be understood that various devices may be added, modified, or omitted.

Hereafter, a "treatment apparatus using RF energy" includes all apparatuses for treating mammals, including humans. A treatment apparatus may include various apparatuses that perform treatment by transmitting RF energy for the purpose of improving the conditions of lesions or tissues. The following embodiment will be described with a focus on an apparatus for treating skin lesions. For example, the apparatus may mean apparatuses that achieve effects such as improvement in wrinkles, tone and textural changes, scars and acne scarring, sagging mucosa, overall rejuvenation, hyperhidrosis laxity, lifting, tightening, and fat reduction by locally heating skin tissues using RF energy. However, it should be noted that the present disclosure is not limited thereto and can be applied to various apparatuses that are used to transmit RF energy to various affected areas, including apparatuses that surgically treat internal organ lesions.

Hereafter, a "tissue" refers to a group of cells constituting various body organs of animals, including humans. The "tissue" includes the skin tissues of the face, neck, arms, legs, and torso, as well as various tissues constituting various internal organs.

Hereafter, a treatment apparatus using RF energy according to an embodiment of the present disclosure is described with reference to the drawings.

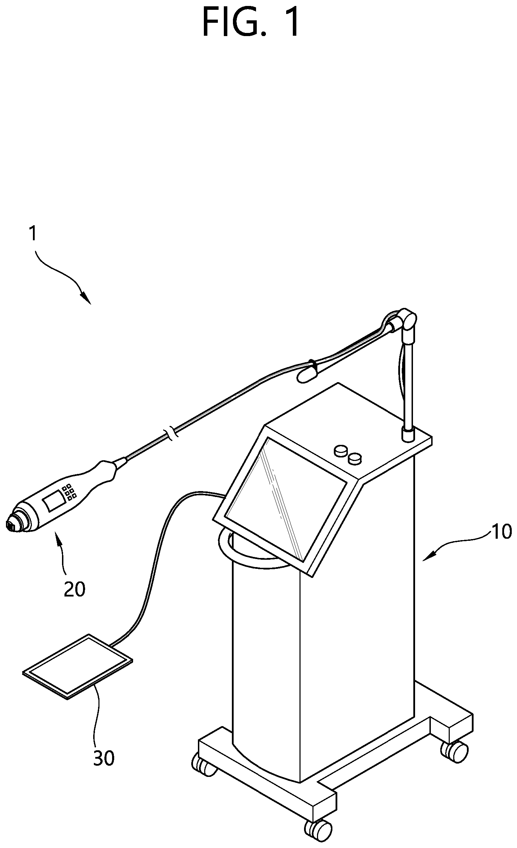

FIG. 1 is a perspective view showing a treatment apparatus using RF energy according to an embodiment of the present disclosure.

As shown in FIG. 1, a treatment apparatus using RF energy according to this embodiment includes a body 10, a handpiece 20 connected with the body 10, and a return electrode pad 30.

The body 10 has various components for operating the treatment apparatus of this embodiment. Various switches and a display unit for setting/performing operations of the treatment apparatus are disposed on the outer surface of the body 10. Further, components such as an RF energy generator 50 and a refrigerant storage may be provided in the body 10.

The handpiece 20 is a component for performing treatment at treatment positions and is designed to be held and used by a user. An electrode 60 that transmits RF energy in contact with the skin surface of a patient is disposed at an end of the handpiece 20. Various controls for operating treatment may be disposed on the outer surface of the handpiece 20, and a conductive path for transmitting RF energy to the electrode 60 and a cooling channel for cooling the electrode are provided in the handpiece 20.

The return electrode pad 30 includes a return electrode. The return electrode is made of a conductive material and is positioned to contact a location on the patient's body opposite a treatment position during treatment. Accordingly, when RF energy is applied, the return electrode forms a path through which RF energy is transmitted to the patient's body together with the electrode of the handpiece.

As shown in FIG. 1, the handpiece 20 and the return electrode pad 30 are connected to the body by connectors, respectively. The connectors may be cables, etc., and each of the connectors is electrically connected to the body, whereby it forms an RF transmission path and is configured to be able to transmit or receive various signals.

In this embodiment, the electrode 60 of the handpiece is configured in a monopolar type having one polarity and is configured to include a separate return electrode pad, but the present disclosure is not limited thereto. As another example, when the electrode 60 of the handpiece is configured is configured in a bipolar type having different polarities, it may not include the return electrode pad.

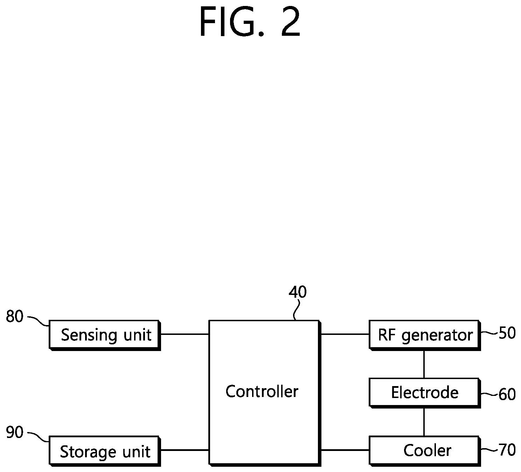

FIG. 2 is a block diagram showing the main configuration of the treatment apparatus shown in FIG. 1.

The RF energy generator 50 generates RF energy that is used for treatment. The RF energy generator 50 generates RF pulses having various parameters, depending on the patient's condition, treatment purpose, treatment area, etc. The parameters may be at least one of output, pulse duration, pulse interval, and frequency. The RF energy generated by the RF energy generator 50 is transmitted to the electrode 60 of the handpiece through the connector and is applied to the skin surface that is in contact with the electrode 60.

The RF energy generator 50 of this embodiment generates RF energy having at least two or more different frequencies. That is, the RF energy generator 50 can selectively generate RF energy having a first frequency and RF energy having a second frequency. In this case, when the frequency range is divided into a first range (2~6MHz), a second range (6~10MHz), and a third range (10~30MHz), the first frequency may be a frequency within the first range and the second frequency may be a frequency within the second range. Alternatively, the first frequency may be a frequency within the first range and the second frequency may be a frequency within the third range. Alternatively, the first frequency may be a frequency within the second range and the second frequency may be a frequency within the third range. When the RF energy generator 50 can generate RF energy having three difference frequencies, the first frequency may be a frequency within the first range, the second frequency may be a frequency within the second range, and the third frequency may be a frequency within the third range.

The RF energy generator 50, depending on a selected mode or the progress of a treatment process, may generate RF energy having any one selected frequency, or may generate RF energy combining RF pulses of a plurality of frequencies while controlling the ratio of the frequencies to be different.

A cooler 70 is a component that cools the electrode 60 of the handpiece. The cooler 70 may be configured in various cooling types, and, as an example, the cooler 70 of this embodiment is configured to cool an electrode by delivering a refrigerant to the rear surface of the electrode. Accordingly, since the electrode 60 that is in contact with skin during treatment is cooled, thermal damage to the skin surface can be prevented during the transmission of RF energy.

In detail, the cooler 70 includes a refrigerant storage that accommodates a refrigerant, a cooling channel that forms a path through which the refrigerant accommodated in the refrigerant storage is delivered to the electrode, and a refrigerant sprayer 152 that sprays the refrigerant, which is delivered through the cooling channel, to the rear surface of the electrode of the handpiece. The refrigerant storage is provided in the body or at a specific location. The cooling channel is connected from the refrigerant storage to the refrigerant sprayer and at least a portion thereof is located inside the handpiece. Further, a valve that adjusts the amount of the refrigerant that is delivered is disposed on the path for delivering the refrigerant, and the on/off operations or the opening and closing amount of the valve can be controlled by a controller 40.

The cooler 70 can be configured to continuously spray a refrigerant to the rear surface of an electrode or to spray a refrigerant to the rear surface of an electrode in the form of pulses at intervals within a predetermined range by controlling a valve, or the like. The cooling performance of the cooler 70 may be adjusted by the amount of a refrigerant that is delivered to the rear surface of an electrode per unit time. The cooling performance may be adjusted by controlling the pressure inside the refrigerant storage, the on/off cycle of the valve, the opening and closing amount of the valve, or the like.

A sensing unit 80 is a component that senses various pieces of information for the operation of the treatment apparatus during or before/after treatment. For example, the sensing unit 80 may be at least one of an impedance sensor that measures the impedance of tissues, a temperature sensor that measures the temperature of an electrode or skin, a contact sensor that senses whether the electrode of the handpiece is in contact with a skin surface, and a movement sensor that senses the movement speed of the handpiece. As an example, a temperature sensor and a contact sensor may be disposed at positions adjacent to the electrode of the handpiece in this embodiment.

A storage unit 90 is a component that stores various pieces of information for treatment and includes a memory device. The storage unit 90 is disposed in the body 10 and may be additionally disposed in the handpiece 20 or a tip module 100. The storage unit 90 can store parameter information about each treatment mode, patient-related information, control information according to sensed conditions, etc. Further, the storage unit 90 may update and record sensed information and information input by a user during treatment. Further, when the handpiece 20 or the tip module 100 has a memory, the storage unit 90 may be configured to store the identification information of the handpiece or the tip module.

The controller 40 is a component that controls the operations of various components of the treatment apparatus such as the RF energy generator 50 and the cooler 70. For example, the controller 40 controls various components in various ways using settings configured by a user through a setting unit or control information stored in the storage unit. The controller receives sensed information from the sensing unit 80 and controls various components using the sensed information. For example, the controller 40 receives a temperature value sensed by a temperature sensor and controls the parameters of RF energy and cooling performance for an electrode on the basis of the temperature value. In this configuration, the controller 40 includes a computing unit and can compute real-time control values from sensing values using a preset algorithm and can control various components on the basis of the real-time control values.

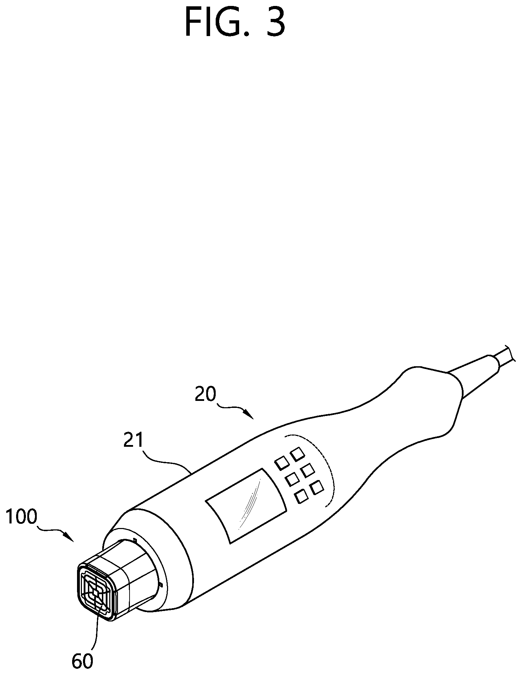

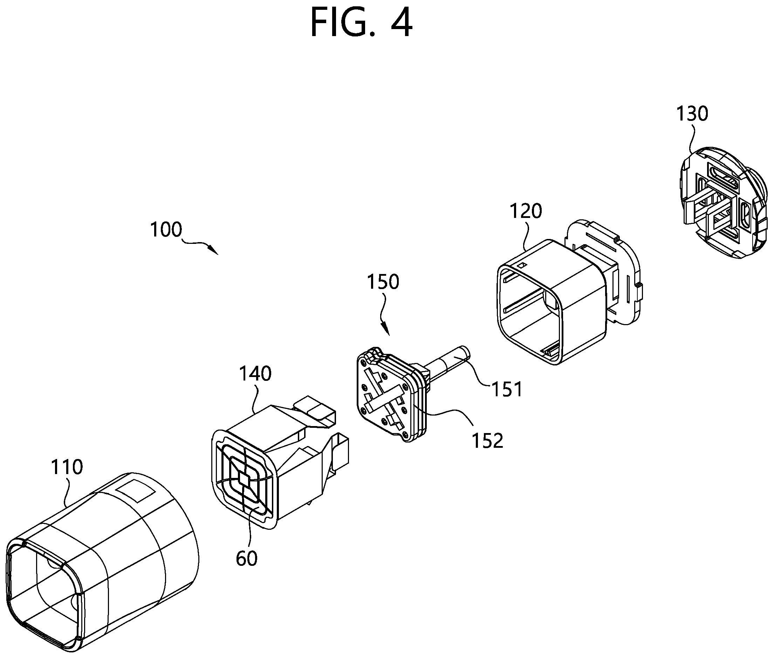

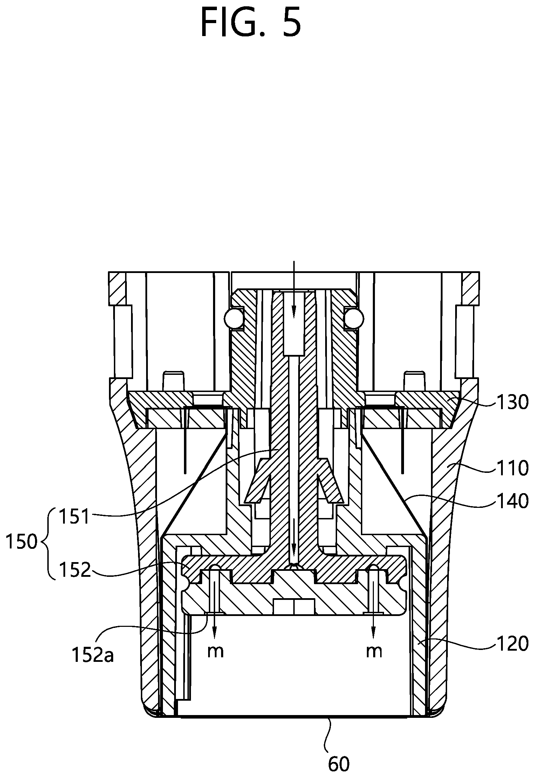

FIG. 3 is a perspective view showing the handpiece shown in FIG. 1. As shown in FIG. 3, the handpiece 2 includes a main body 21 and a tip module 100. An end of the main body 21 is connected with a connector, and various components for performing treatment operations such as an RF transmission circuit and a cooling channel are disposed in the main body 21. Controls and a display unit such as a display may be disposed on the outer surface of the main body 21. The tip module 100 has the electrode 60 for transmitting RF energy in contact with skin and is detachably coupled to an end of the main body 21. The tip module 100 has a circuit for transmitting RF energy to the electrode and a cooling structure for cooling the electrode. Hereafter, the structure of the tip module is described in more detail with reference to FIG. 4 and FIG. 5.

FIG. 4 is an exploded perspective view showing the main configuration of the tip module shown in FIG. 3 and FIG. 5 is a cross-sectional view showing the main configuration of the tip module shown in FIG. 3. Referring to FIG. 4 and FIG. 5, the tip module includes a tip housing 110, an electrode module 140, a cooling channel block 150, an internal case 120, and a rear cover 130.

The tip housing 110 and the internal case 120 are combined with each other, thereby supporting the electrode module 140. The cooling channel block 150 is disposed in the internal case 120. The rear cover 130 is coupled to the rear surface of the tip housing 110 with the electrode module 140, the internal case 120, and the cooling channel block 150 disposed in the tip housing 110. The tip housing 110 or the rear cover 130 has a coupling structure for fastening to the end portion of the main body 21 of the handpiece.

As shown in FIG. 4, the electrode module 140 is a flexible substrate that can be folded and has electric devices and a circuit for electrically forming them. The electrode 60 is disposed on the front of the electrode module (when the electrode module is folded) and exposed through an opening of the tip housing 110 to come in contact with a skin surface. The electrode 60 includes a conductive layer formed on the flexible substrate and the conductive layer is configured to be covered by a dielectric layer. Accordingly, in treatment, the conductive layer of the electrode is brought in contact with skin through the dielectric layer, and when RF energy is applied to the electrode, the electrode transmits the RF energy to skin tissues in a capacitively coupled state to the skin tissues by the dielectric layer.

Meanwhile, as described above, the temperature sensor and the contact sensor of the sensing unit are disposed at positions adjacent to the electrode 60 of the electrode module 140 and measure the temperature of the electrode or a skin surface and sense whether the electrode comes in contact with skin. Further, the electrode module 140 further includes a memory and the memory can store information of the tip module such as the kind of the electrode, the size of the electrode, the pattern of the electrode, and the size of the cooling space. The electrode module 140 has a conductive lead that is connected with the electrode, the sensors, and the memory described above and extends rearward. A terminal formed at the end portion of the conductive lead is exposed rearward with the rear cover 130 coupled thereto and is electrically connected with the RF circuit at the main body 21 of the handpiece when the tip module 100 is coupled.

Further, the cooling channel block 150 has a cooling channel that forms a path through which a refrigerant is delivered, and a cooling sprayer 152 for spraying the refrigerant delivered through the cooling channel to the rear surface of the electrode. The cooling channel 151 forms a passage extending rearward from the cooling sprayer, and the end portion of the cooling channel 151 is exposed rearward from rear cover 130 when the tip module 100 is assembled. Accordingly, when the tip module 100 is combined to the main body 21 of the handpiece, the end portion of the cooling channel 151 is coupled to the cooling channel at the main body 21, thereby forming a path through which the refrigerant delivered from the refrigerant storage is delivered. The refrigerant sprayer 152 is disposed in front of the cooling channel 151 and has a plurality of diverging passages and spray holes formed at the ends of the passages, respectively. Accordingly, the refrigerant delivered through the cooling channel is separated into the plurality of passages while passing through the cooling sprayer 152 and then sprayed to the cooling space on the rear surface of the electrode through the spray holes.

The tip module 100 having this structure, as described above, is detachably coupled to the end portion of the main body of the handpiece. When the tip module 100 is coupled, the controller 40 is provided with information of the tip module from the memory of the tip module 100 and controls the RF energy generator 50 and the cooler 70 in consideration of the information, thereby performing a process of transmitting RF energy to the electrode of the tip module 100 and cooling the electrode. Further, the tip module 100 may be a consumable and can be replaced with a new tip module 100 when a new patient is treated or the allowed number of uses is exceeded.

As described above, the treatment apparatus 1 using RF energy performs treatment by applying RF energy to a skin surface through the electrode 60. In this configuration, the temperature of the electrode 60 at the start of treatment is likely to vary, depending on the treatment environment and the condition of a patient when there is on specific control. In detail, the initial temperature of the electrode 60 may be influenced by the room temperature of a treatment place and may be influenced by the temperature of the skin surface of a patient that is in contact with the electrode. However, the electrode 60 forms a path through which RF energy is transmitted in treatment and the temperature of the electrode influences the electrical property of the electrode. Accordingly, when the temperature of the electrode 60 is changed even though RF energy with the same parameters is applied, different treatment results may be obtained.

In order to overcome this concern, the treatment apparatus 1 according to this embodiment is configured to actively control the temperature of the electrode 60 before starting treatment so that treatment is started with the temperature of the electrode 60 maintained at a predetermined level. In general, treatment apparatuses 1 cool an electrode or a skin surface to prevent damage to the skin surface while RF energy is applied. However, the treatment apparatus of this embodiment additionally performs a step of preliminarily cooling the electrode 60 before RF energy is applied to the electrode 60.

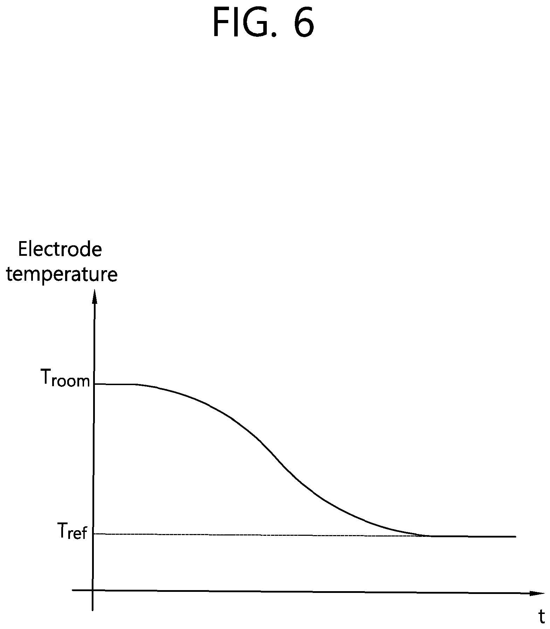

FIG. 6 is a graph showing temperature change of an electrode according to precooling in FIG. 3. As described above, the controller 40 controls the cooler 70 to perform precooling on the electrode 60. Since control for precooling is performed, the electrode 60 is cooled to a preset reference temperature from the initial temperature and is maintained at the reference temperature T_{ref}. In the precooling step of the electrode, the initial temperature of the electrode may be a temperature Tᵣₒₒₘ corresponding to the room temperature of the space where treatment is performed. Further, the preset reference temperature T_{ref} may be a temperature lower than the room temperature and may be a value within the range of 0 to 25°C, in more detail, a value within the range of 5 to 20°C. As an example, the reference temperature of this embodiment may be 20°C.

In this case, the reference temperature T_{ref} may have one value in all treatments, but the reference temperature can be differently set by any one of the setting by a user, a treatment mode, the location of target tissues, power of RF energy, a frequency, and a pulse pattern. For example, in the case of a treatment mod targeting tissues (e.g., the dermis) adjacent to a skin surface, the reference temperature T_{ref} may be set lower in comparison to a treatment mode targeting tissues located deeper from the skin surface (e.g., subcutaneous fat) as a target location. As another example, the reference temperature T_{ref} may be set relatively high in the case of treatment using RF energy having a frequency (e.g., 2 ~ 6MHz) with an excellent skin penetration characteristic and having high intensity, and the reference temperature T_{ref} may be set relatively low in the case of treatment using RF energy having a frequency (e.g., 6 ~ 10MHz) with a relatively low skin penetration characteristic and having high intensity. As another example, when treatment is performed with high-power RF energy, the reference temperature T_{ref} may be set low, as compared with when treatment is performed with low-power RF energy.

In this case, the controller 40 performs precooling on the basis of the information of the tip module stored in the memory of the tip module 100. The stored tip module information may be at least any one piece of information among the identification number of the tip module, the model of the tip module, the size of the tip module, the size of the electrode, or the size of the cooling space. Accordingly, the controller 40 can cool the electrode by sensing the information of the tip module 100 fastened to the handpiece and spraying a refrigerant by the amount corresponding to the tip module. For example, when the information of the tip module sensed through the memory corresponds to a large tip module, the controller can perform control to precool the electrode by spraying a relatively large amount of refrigerant. Further, when the information corresponds to a small tip module, the controller can perform control to precool the electrode by spraying a relatively small amount of refrigerant. Further, when precooling is performed, the controller can monitor in real time the temperature of the electrode or the skin surface that is in contact with the electrode from the temperature sensor of the tip module and can perform feedback control such that the temperature of the electrode is maintained at the reference temperature T_{ref}.

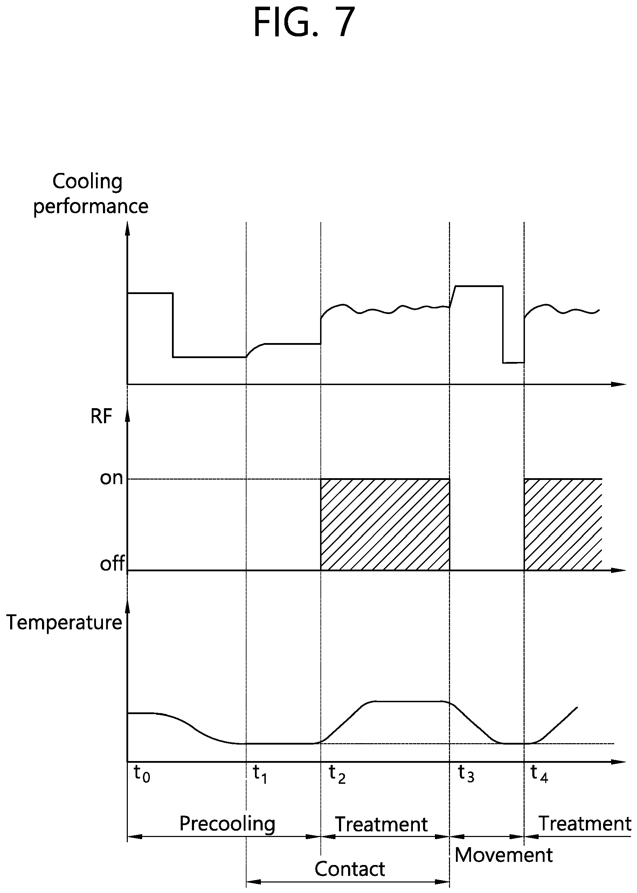

Hereafter, a cooling pattern and an RF application pattern according to a treatment process are described in detail with reference to FIG. 7. FIG. 7 is a graph showing cooling and RF application patterns that are applied to an electrode in accordance with a treatment process. In FIG. 7, the horizontal axis represents a time axis of a treatment process and the vertical axes represent the cooling performance of the cooler, the power of RF energy, and the temperature value sensed by the temperature sensor, respectively.

As shown in FIG. 7, the starting time point tₒ of precooling of the electrode is earlier than the RF application time point t₂ of the electrode and the time point t₁ at which the electrode comes in contact with skin. When preset conditions are satisfied, the controller 40 starts precooling of the electrode and it is started regardless of the RF application time point of the electrode or the time point when the electrode comes in contact with skin. That is, the interval between the starting time point of precooling of the electrode and the RF application time point of the electrode and the interval between the starting time point of precooling of the electrode and the time point when the electrode comes in contact with skin are not constant and can be varied in accordance with the use aspects of users.

As an example, the treatment apparatus may be configured such that precooling of the electrode is automatically started when the tip module 100 is fastened to the handpiece. In this case, cooling is continued with the tip module fastened to the handpiece, whereby the reference temperature T_{ref} can be maintained. As another example, when a predetermined condition is satisfied with the tip module 100 fastened to the handpiece 20, precooling can be started. In this case, the predetermined condition may be that a user instructs precooling through manipulation or that a user sets a treatment mode.

During the precooling step of the electrode 60, the controller 40 preliminarily cools the electrode by controlling the cooler 70 and a user brings the cooled electrode 60 in contact with a skin surface corresponding to a treatment position. In this case, the temperature of the skin surface may be higher than the temperature of the electrode. Even with the electrode in contact with the skin, the controller 40 performs feedback control in real time on the basis of the temperature value sensed by the temperature sensor to maintain the temperature of the electrode 60 at the preset reference temperature T_{ref}. Accordingly, the cooling performance of the cooler with the electrode in contact with skin may be slightly increased to compensate for an increase by the skin temperature.

In this case, the skin surface is cooled by contact with the precooled electrode before RF application, so it is possible to reduce the patient's pain and minimize thermal damage to the skin surface in treatment. Further, since the electrode 60 and the skin surface are in contact over a predetermined time, the skin surface may be cooled to the reference temperature of the electrode. In general, the skin temperatures of patients are different, depending on the conditions and characteristics of the patients. The temperature of a skin surface influences the electrical characteristic of tissues, and, considering that the treatment using RF energy is a thermal treatment type, the difference of initial temperatures of skin may influence the treatment result. Accordingly, when a skin surface is cooled in advance to a predetermined temperature by the precooled electrode and then treatment is performed, as in this embodiment, it is possible to expect a uniform treatment effect despite the differences in the treatment environment and patients' characteristics.

Meanwhile, since RF energy is applied with the electrode 60 and skin in contact, treatment is performed on the skin tissues. During treatment, the controller controls the cooler 70 on the basis of the temperature value sensed by the temperature sensor, thereby cooling the electrode and the skin surface. Even though heat is generated on the electrode and the skin surface due to application of the RF energy, the temperatures of the electrode and the skin surface can be maintained within an appropriate range by the cooling control. However, the appropriate range may be a temperature range higher than the reference temperature of the precooling step and the cooling performance may also increase in comparison to the level immediately before the RF energy is applied (a maintenance step of the precooling step).

Further, when the treatment at the position is finished t₃, the user separates the electrode from the treatment position and moves the handpiece 20 to the next treatment position. In this case, the controller 40 controls the cooler 70 to be able to preliminarily cool the electrode while the handpiece 20 is moved so that the temperature of the electrode increased during the treatment can be cooled back to the reference temperature. Accordingly, treatment can also be started for the next target position with the electrode maintained at the preset reference temperature t₄.

As described above, the electrode 60 is cooled through a precooling step before treatment, the main cooling step during treatment, and the precooling step during movement, and this cooling process is continuously performed while adjusting the cooling performance. In this case, 'continuously performed' not only refers to continuously providing a refrigerant to the electrode during the steps described above but also includes continuously applying refrigerant pulses at predetermined intervals. Therefore, according to this embodiment, the temperature of the electrode is continuously and actively managed without any periods of neglect while the precooling step and the main cooling step are performed.

For this cooling control, the cooler 70 of this embodiment uses a proportional control valve as a valve for opening and closing the cooling channel. The proportional control valve is configured to be able to adjust the opening and closing amount by control of the controller 40. In the related art, valves having a constant opening and closing amount were used, and when additional cooling was required, control was performed by increasing the pulse width of cooling pulses or the number of cooling pulses. However, this is a method of essentially increasing the cooling time and there is no significant difference in the amount of a refrigerant that is delivered per unit time, so it is disadvantageous for a continuous cooling process and real-time feedback control, as in this embodiment. In contrast, when a proportional control valve is used, as in this embodiment, it is possible to adjust the cooling performance in real time in accordance with the cooling process by adjusting the opening and closing amount while applying cooling pulses to have predetermined pulse width and intervals.

For this cooling control, the valve of the cooler according to this embodiment is configured to be able to not only open and close the cooling channel but also adjust the cooling performance by adjusting the opening and closing amount when opening the channel. In treatment apparatuses of the related art, valves that simply perform opening and closing operations and form a channel with a constant opening and closing amount were used. Accordingly, when additional cooling was required, control was performed by increasing the pulse width of cooling pulses or the number of cooling pulses that were applied. However, this is a method of essentially increasing the cooling time and there is no significant difference in the amount of a refrigerant that is delivered per unit time, so it is disadvantageous for a continuous cooling process and feedback control based on sensed information, as in this embodiment. In contrast, when a valve that can adjust the opening and closing amount of a channel is used, as in this embodiment, it is possible to adjust the cooling performance in real time in accordance with the cooling process by changing the amount of a refrigerant that is delivered per unit time by adjusting the cross-sectional area of the channel while applying cooling pulses with a predetermined pulse width at predetermined intervals.

As an example, the valve according to this embodiment can operate in at least three or more modes. The valve can operate to open the channel by a first cross-sectional area in a first mode (e.g., full opening), open the channel by a second cross-sectional area in a second mode (e.g., half opening), and fully close the channel in a third mode. In this case, even though cooling pulses are applied with a predetermined pulse width at predetermined intervals, it is possible to variously adjust the cooling performance by controlling the opening and closing amount of the valve. An example of operating in three modes was described above, this is for the convenience of description and it is also possible to configure the valve to be able to operate in three or more modes having different opening and closing amounts or linearly adjust the opening and closing amount in accordance with signals. The valve of the cooler according to this embodiment is, for example, a proportional control valve. However, in addition to this, it is also possible to configure a valve that adjusts the opening and closing amount of a channel using a servo valve, a solenoid valve, a shutter, a motor, etc.

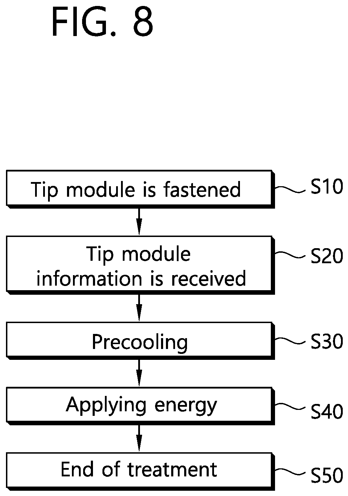

FIG. 8 is a flowchart showing a method for controlling the treatment apparatus using RF energy according to an embodiment. Hereafter, an example of the method for controlling the treatment apparatus using RF energy of this embodiment is described in detail using FIG. 8.

In order to perform treatment, a user fastens the tip module 100 to the main body of the handpiece 20 (S10). The controller 40 detects the fastening of the tip module and receives information of the tip module from the memory of the tip module (S20). As described above, the information of the tip module includes at least any one of the identification number of the tip module, the model of the tip module, the size of the tip module, the size of the electrode, or the size of the cooling space.

When receiving the information of the tip module, the controller 40 controls the cooler 70, thereby performing the precooling step (S30). The precooling step may be automatically started when the information of the tip module is received after the tip module 100 is fastened. Alternatively, the precooling step may be started by an operation of instructing precooling by the user after the information of the tip module is received. Accordingly, the precooling step is started before treatment using RF energy is performed, and is performed before the electrode of the handpiece is brought in contact with a skin surface.

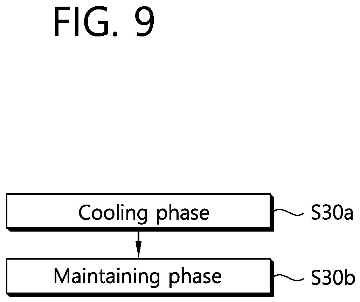

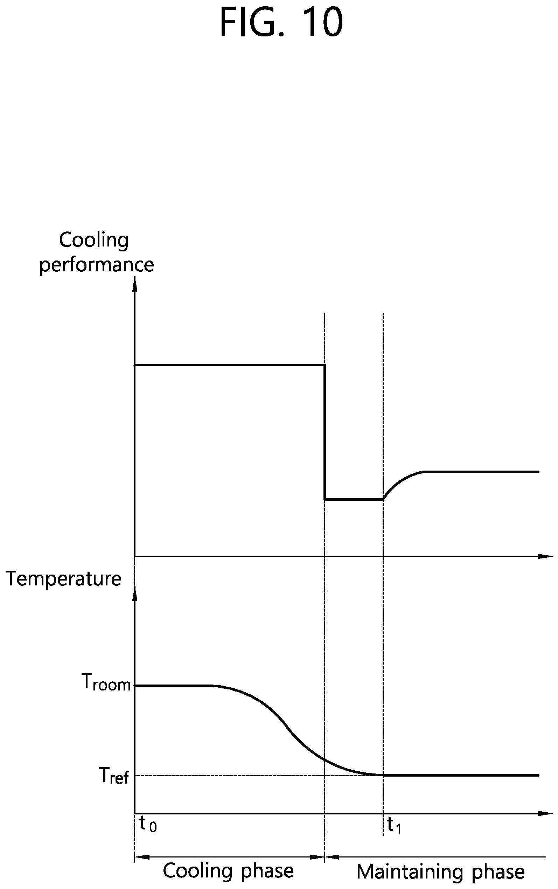

FIG. 9 is a flowchart showing the detailed steps of the precooling step shown in FIG. 8 and FIG. 10 is a graph showing a cooling pattern and temperature change in the precooling step shown in FIG. 7.

The precooling step S30 is composed of at least two phases including a cooling phase S30a and a maintaining phase S30b. The cooling phase S30a is a phase that rapidly decreases the temperature of the electrode to a level close to the preset reference temperature T_{ref} and the maintaining phase S30b is a phase that performs control to maintain the temperature of the electrode cooled through the cooling phase at the reference temperature T_{ref}.

As shown in FIG. 10, in the cooling phase S30a, a relatively large amount of refrigerant is delivered to the electrode per unit time to decrease the initial temperature of the electrode that corresponds to the room temperature. In the cooling phase S30a, the controller 40 determines necessary cooling performance on the basis of the set reference temperature, the current temperature (room temperature or sensed temperature), and the tip module information transmitted from the memory of the tip module, and controls the cooler 70 on the basis of the cooling performance. For example, when the difference between the reference temperature and the current temperature is large or the size of the tip module is large, the controller performs control to increase the cooling performance by increasing the opening and closing amount of the proportional control valve or to increase the duration of the cooling phase.

The cooling phase S30a may also be controlled in a feedback control type based on a temperature sensor. However, in this embodiment, the storage unit stores control parameters for the cooling phase according to the reference temperature, the current temperature, and the tip module information and the controller performs the cooling phase by controlling the cooler in accordance with the stored parameters.

In the maintaining phase S30b, a relatively small amount of refrigerant is delivered to the electrode per unit time to maintain the temperature of the cooled temperature at a level close to the reference temperature T_{ref}. In the maintaining phase S30b, the cooler may be controlled in a feedback control type based on a temperature, and as in the cooling phase described above, it may be controlled in accordance with the control parameters stored in the storage unit.

However, when a user brings the electrode of the handpiece in contact with the skin surface of the user before RF application, the temperature of the skin surface may influence the temperature of the electrode. Accordingly, when contact of the electrode with the skin surface is sensed by a contact sensor in the maintaining phase S30b (t₁), the controller 40 controls the cooler 70 so that the temperature of the electrode can be maintained at the reference temperature T_{ref}. In this case, the controller 40 performs feedback control on the cooler 70 with the electrode in contact with the skin on the basis of the temperature value sensed by the temperature sensor. Accordingly, the cooler 70 can be controlled to operate with high cooling performance when the electrode is in contact with the skin in the maintaining phase S30b, as compared with when the electrode is not in contact with the skin. Further, since the maintaining phase continues for a predetermined time with the electrode in contact with the skin, not only the temperature of the electrode but also the temperature of the skin surface can be decreased to temperature close to the reference temperature.

Referring to FIG. 8 again, when the precooling step is performed by the method described above, the controller 40 performs a step of applying RF energy to the electrode for treatment (S40). The controller 40 controls the RF energy generator and the RF circuit on the basis of a set treatment mode, whereby RF energy is transmitted in a preset pattern. While the RF energy is transmitted, the cooler 70 performs the main cooling step, and in this step, the controller 40 controls the cooler 70 such that the temperature of the skin surface maintains an appropriate range. In this case, the appropriate range is a temperature range at a level where damage to the skin surface is not generated, and the controller 40 monitors in real time the temperature sensed by the temperature sensor while the RF energy is applied, and performs feedback control on the cooling process of the cooler on the basis of the sensed value.

In this case, the cooler 70 of this embodiment includes a proportional control valve and the controller controls the operation of the cooler 70 in consideration of not only the temperature value sensed by the temperature sensor but also temperature change per hour. For example, even though the temperature itself sensed by the temperature sensor maintains the appropriate range, when an increase of the rate of temperature change exceeding a preset range is sensed, the controller 40 performs control to increase the opening and closing amount of the proportional control valve. In this case, the control of the cooler 70 does not respond as a result of a temperature increase and it is possible to predict a temperature increase and respond proactively, whereby safe treatment is possible.

When RF energy at a target level is applied to the treatment position through the step described above, the energy application step is finished and treatment for the position is finished. However, FIG. 9 shows steps through which treatment is normally finished, but when a problem occurs during treatment, the treatment may be abnormally finished for the safety of the patient.

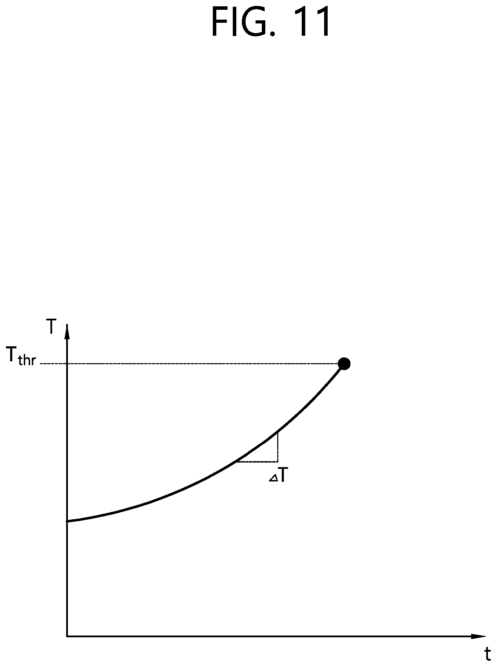

FIG. 11 is a graph exemplarily showing temperature sensed by a temperature sensor. As described above, in the treatment step, the controller 40 controls the cooler 70 on the basis of the temperature value sensed by the temperature sensor and the rate of temperature change. However, when the ideally sensed temperature exceeds a threshold value Tₜₕᵣ or the rate of temperature change exceeds a threshold rate of change, treatment does not proceed further and is urgently terminated. In this case, the controller 40 performs cooling with maximum performance by maximizing the opening and closing amount of the proportional control valve and stops transmission of RF energy to the electrode by controlling the RF energy generator 50.

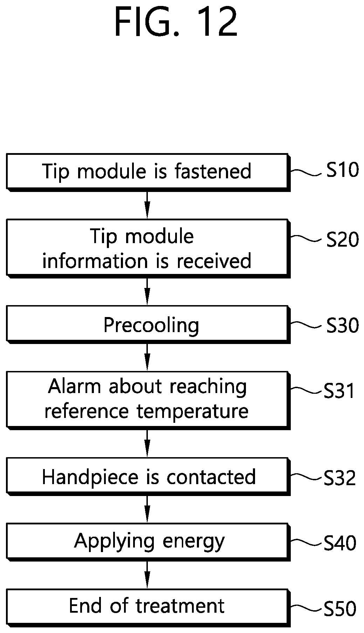

FIG. 12 is a flowchart showing a method for controlling the treatment apparatus using RF energy according to another embodiment. The embodiment shown in FIG. 12 performs the same steps as those of the embodiments shown in FIG. 8 described above and may additionally perform some steps. Accordingly, the steps that are the same as the steps shown in FIG. 8 are referred to by the description of FIG. 8.

This embodiment further performs a step in which when the temperature of the electrode is decreased to the reference temperature T_{ref} by the precooling step, the controller informs a user of it (S31). When the reference temperature is reached it can be determined on the basis of the value sensed by the temperature sensor. The time point when the reference temperature is reached may be the same as the time point when the cooling phase described above is finished, and, considering thermal conductivity delay, it may be a time point that is slightly later than the time point when the cooling phase is finished. The controller 40 can informs a user of the time point when the reference temperature is reached using an alarm sound, etc., and the user can bring the handpiece in contact with skin and perform treatment after hearing the alarm sound (S32).

Meanwhile, as described above, when the electrode cooled to the reference temperature is brought in contact with skin, the skin surface is cooled. Further, when a predetermined time has passed in the contact state, the skin surface is cooled to temperature corresponding to the reference temperature, whereby it is possible to perform treatment with the temperature of the skin surface maintained at the same level. Accordingly, though not specifically shown in FIG. 12, when the electrode of the handpiece is brought in contact with skin (S32), the controller 40 may further perform a step of measuring the time that has passed from the time point of contact and informing a user of the time point when a predetermined time has passed using an alarm sound. Accordingly, the user recognizes that the predetermined time has passed, and can perform treatment after ensuring that the skin surface has been precooled in advance to predetermined temperature, so safe and uniform treatment can be performed. However, without informing a user of the time point when the predetermined time has passed, the controller 40 may perform control to apply RF energy after a predetermined time passes from the time point of skin contact even though the user instructs application of RF energy through controls.

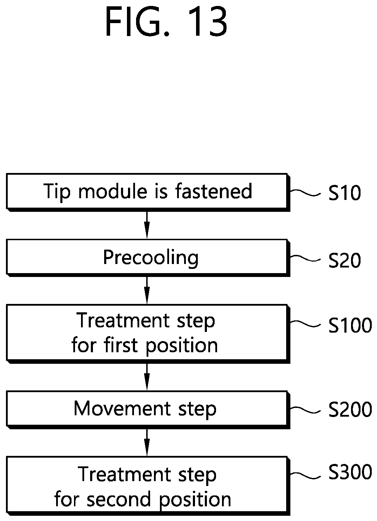

FIG. 13 is a flowchart showing a treatment method using the treatment apparatus using RF energy according to another embodiment. A control method for one treatment position was mainly described in the embodiments of FIG. 8 and FIG. 12 described above, but FIG. 13 shows a method of performing treatment on a plurality of treatment positions while moving the handpiece.

As shown in FIG. 13, when the tip module is fastened, the step of preliminarily cooling the electrode is performed and a step of treating a first position is performed with the electrode in contact with the first treatment position. Up to this step, the method is performed in the same manner as the steps shown in FIG. 8 or FIG. 12, so the detailed description of the steps is referred to the above description.

When treatment for the first position is finished through the step (S100), the user moves the handpiece 20 to a second position (S200) and performs the treatment steps for the second position (S300). However, upon completion of the treatment of the first position, the temperature of the electrode is higher than the reference temperature due to application of RF energy during the treatment for the first position. Accordingly, in this embodiment, a step of preliminarily cooling again the electrode to the reference temperature before treatment for the second position is performed, and this step may be performed when moving the handpiece (see FIG. 7).

In detail, the precooling step after completing treatment for the first position may be started at the time point when application of RF energy is finished. Alternatively, the precooling step may be started at the time point when the electrode is separated from the skin surface of the first position. Alternatively, the precooling step may be started at the time point when movement is sensed by a movement sensor after finishing treatment for the first position.

In the precooling step that is performed in the movement step, the controller 40 can control the cooler 70 on the basis of the sensed temperature of the electrode, the reference temperature, and the information of the tip module. In detail, the controller, as described with reference to FIG. 9 and FIG. 10, can perform this step by dividing it into the cooling phase S30a and the maintaining phase S30b.

According to this embodiment, it is possible to perform treatment for the second step with the temperature of the electrode decreased back to the reference temperature T_{ref} after finishing treatment for the first position. The steps of treating a first position and a second position are mainly described with reference to FIG. 13, but it is possible to treat more positions in the manner of performing treatment after a precooling step during movement. Accordingly, even though treatment is performed on a plurality of positions, the treatment is performed with the temperature of the electrode maintained at a predetermined level, so it is possible to achieve uniform treatment effect.

The treatment field that uses a monopolar type of electrode was mainly described in the above embodiments, but the present disclosure is not limited thereto and can be applied to a treatment field that uses a bipolar type of electrode. Further, the aforementioned content can be applied to the field of treating skin tissues in various areas such as the face, neck, abdomen, and thighs using RF energy.

Embodiments of the present disclosure were described above in detail, but the present disclosure is not limited to the embodiments described above. It should be noted that those skilled in the art can change and modify the present disclosure in various ways without departing from the range of technical features defined in claims.

## Claims

1. A treatment apparatus using RF energy, comprising:
a handpiece having an electrode configured to transmit RF energy in contact with a skin surface;
an RF energy generator configured to generate RF energy that is transmitted to the electrode;
a cooler configured to cool the electrode; and
a controller configured to control operation of the RF energy generator and the cooler and control the cooler to preliminarily cool the electrode before RF energy is applied to the electrode.

2. The treatment apparatus of claim 1, wherein the controller performs control to preliminarily cool the electrode such that the electrode forms a preset reference temperature without RF energy applied.

3. The treatment apparatus of claim 2, wherein the reference temperature is temperature lower than room temperature.

4. The treatment apparatus of claim 2, wherein the reference temperature has a value within a range of 0 ~ 25°C.

5. The treatment apparatus of claim 2, wherein the reference temperature can be adjusted in accordance with a treatment mode or setting by a user.

6. The treatment apparatus of claim 1, wherein the controller performs control to preliminarily cool the electrode regardless of a time point when the electrode is brought in contact with the skin surface or a time point when RF energy is applied to the electrode.

7. The treatment apparatus of claim 1, wherein the controller controls the cooler to cool the electrode while RF energy is applied through the electrode, and
the cooler is controlled such that a process of preliminarily cooling the electrode and a process of cooling the electrode while the RF energy is applied are continuously performed.

8. The treatment apparatus of claim 1, wherein the handpiece further comprises a tip module having the electrode disposed on one surface thereof and detachably coupled to an end portion of a main body of the handpiece, and
when the tip module is coupled to the main body of the handpiece, the controller performs control to automatically preliminarily cool the electrode.

9. The treatment apparatus of claim 1, wherein the handpiece further comprises a tip module having the electrode disposed on one surface thereof and detachably coupled to an end portion of a main body of the handpiece, and
the tip module further comprises a memory configured to store information of the tip module and the controller performs control to preliminarily cool the electrode on the basis of the information of the tip module stored in the memory.

10. The treatment apparatus of claim 1, wherein the cooler comprises a refrigerant storage in which a refrigerant is accommodated, a cooling channel at least partially located in the handpiece and configured to form a path through which the refrigerant accommodated in the refrigerant storage is transmitted to the electrode, and a valve configured to adjust the amount of a refrigerant that is delivered through the cooling channel; and
the valve is configured to be able to adjust the opening and closing amount of the channel to adjust cooling performance.

11. The treatment apparatus of claim 1, wherein the handpiece further comprises a contact sensor disposed adjacent to the electrode and configured to sense a contact state of skin of a patient and the electrode, and
the controller performs control to transmit the RF energy after a preset time has passed from a time point when the electrode has been brought in contact with a skin surface of a patient.

12. The treatment apparatus of claim 1, wherein the controller controls the cooler to preliminarily cool the electrode while a user moves the handpiece to a second position after treating a first position.

13. A method of controlling a treatment apparatus using RF energy, the method comprising:
sensing that a tip module having an electrode configured to transmit RF energy in contact with a skin surface is fastened to an end of a handpiece;
preliminarily cooling the electrode by means of a cooler before RF energy is transmitted through the electrode; and
applying RF energy generated by an RF energy generator to the cooled electrode.

14. The method of claim 13, wherein the preliminary cooling cools the electrode to form a preset temperature without the electrode in contact with a skin surface.

15. The method of claim 13, wherein the cooler is controlled to continuously cool the electrode until treatment is finished from the preliminary cooling of the electrode.

16. The method of claim 13, wherein when coupling of the tip module to the handpiece is sensed, the controller controls the cooler to automatically and preliminarily cool the electrode.

17. The method of claim 13, further comprising receiving information of the tip module from a memory disposed in the tip module when coupling of the tip module to the handpiece is sensed,
wherein a controller controls the cooler to preliminarily cool the electrode on the basis of the received information of the tip module.

18. The method of claim 13, wherein the applying of RF energy is controlled to apply RF energy after a preset time has passed from a time point when the electrode has been brought in contact with a skin surface of a patient.

19. The method of claim 13, further comprising cooling the electrode heated due to application of the RF energy while moving the handpiece to another position after the RF energy is applied.

20. A treatment method using RF energy, the treatment method comprising:
preliminarily cooling an electrode configured to transmit RF energy in contact with a skin surface, to a preset temperature; and
applying RF energy by contacting the electrode cooled to a preset temperature to the skin surface.
